# NEUE EUROPÄISCHE PATENTSCHRIFT

(11) **EP 3 137 041 B2**
(45) Veröffentlichungstag und Bekanntmachung des Hinweises auf die Entscheidung über den Einspruch: **18.01.2023**
(45) Hinweis auf die Patenterteilung: 06.11.2019
(21) Anmeldenummer: 15714837.0
(22) Anmeldetag: 10.04.2015
(51) Int. Cl.: A61K 8/24, A61K 8/36, A61K 8/362, A61K 8/365, A61K 8/49, A61K 8/55, A61Q 17/04

(54) **SONNENSCHUTZMITTEL MIT REDUZIERTER NEIGUNG ZUR TEXTILVERFLECKUNG**
SUNSCREEN HAVING REDUCED TENDENCY TO STAIN TEXTILES
PRODUIT DE PROTECTION SOLAIRE AYANT UNE TENDANCE RÉDUITE À FORMER DES TACHES SUR LES TEXTILES

(30) Priorität: 28.04.2014 DE 102014207919
(43) Veröffentlichungstag der Anmeldung: 08.03.2017
(73) Patentinhaber: Beiersdorf AG, 20253 Hamburg (DE)
(72) Erfinder: WEINERT, Katrin, 22763 Hamburg (DE); BORCHERS, Kathrin, 21614 Buxtehude (DE); SCHADE, Tatjana, 25866 Mildstedt/Rosendahl (DE); BLECKMANN, Andreas, 22926 Ahrensburg (DE)
(74) Vertreter: Beiersdorf AG
(86) Internationale Anmeldenummer: PCT/EP2015/057808
(87) Internationale Veröffentlichungsnummer: WO 2015/165711

(56) Entgegenhaltungen:
- EP-A1- 1 454 619
- EP-A1- 1 541 152
- EP-A1- 2 543 356
- DE-A1-102008 018 786
- JP-A- 2005 206 473
- JP-A- 2011 236 199
- US-A1- 2005 238 679
- US-A1- 2007 092 459
- US-A1- 2008 115 846
- US-A1- 2012 156 149
- US-A1- 2013 104 319
- US-A1- 2013 309 185

## Beschreibung

Die vorliegende Erfindung betrifft eine kosmetische Zubereitung enthaltend 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) und einen oder mehrere Komplexbildner gewählt aus der Gruppe der Phosphonsäuren, Phosphorsäuren und Carbonsäuren mit weniger als 2 Stickstoffatomen und/oder deren Alkalisalzeund/ oder deren Amin-N-Oxide.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch..

Zwar kennt der Fachmann die WO2011/101250, doch konnte diese Schrift nicht den Weg zur vorliegenden Erfindung weisen.

WO 2012078961 beschreibt chelatisierende Polymere zur Photostabilisierung von Avobenzone und Verminderung der Ausbildung von farbigen Komplexen bei Waschen in hartem Wasser, doch konnte diese Schrift ebenfalls nicht den Weg zur vorliegenden Erfindung weisen.

Darüber hinaus kennt der Fachmann grundsätzlich kosmetische Zubereitungen mit 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) und dem Komplexbildner EDTA. Die Wirkung zur Fleckenreduktion von EDTA ist im alkalischen Medium, wie es im Waschwasser vorliegt, jedoch begrenzt.

Nicht zuletzt kennt der Fachmann die US 2007/092459, US 2005/238679, JP 2005/206473, JP 2011/236199, EP 1454619, EP 1541152, DE 102008018786US, 2008/115846, US 2013-309185 und US 2012/156149, die ebenfalls nicht den Weg zur vorliegenden Erfindung weisen konnten.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche Breitbandfilter wie, Bis-Ethylhexyloxyphenol Methoxyphenyl Triazine zu entwickeln, welche sich leichter aus den mit der Zubereitung kontaminierten Textilen herauswaschen lassen.

Überraschend gelöst wird die Aufgabe durch eine kosmetische Zubereitung gemäß Anspruch 1, ein Verfahren gemäß Anspruch 9 und eine Verwendung gemäß Anspruch 10.

Es ist erfindungsgemäß vorteilhaft, wenn die erfindungsgemäße Zubereitung 2,4-Bis-{[4-(2-ethyl-hexyloxy)-2-hydroxy]-phenyl}-6-(4-methoxyphenyl)-1,3,5-triazin in einer Menge von 0,01 bis 10 Gewichts-%, bezogen auf das Gesamtgewicht der Zubereitung, enthält.

Es ist erfindungsgemäß bevorzugt, wenn die erfindungsgemäße Zubereitung 2,4-Bis-{[4-(2-ethyl-hexyloxy)-2-hydroxy]-phenyl}-6-(4-methoxyphenyl)-1,3,5-triazin in einer Menge von 2 bis 5 Gewichts-%, bezogen auf das Gesamtgewicht der Zubereitung, enthält.

Als erfindungsgemäß vorteilhafte Alkalisalze gelten dabei Natrium- und Kaliumsalze, wobei die Natriumsalze erfindungsgemäß bevorzugt werden.

Es ist erfindungsgemäß vorteilhaft, wenn die erfindungsgemäße Zubereitung in Form einer Emulsion vorliegt. Erfindungsgemäß bevorzugt ist es in einem solchen Fall, wenn die Zubereitung in Form einer Öl-in-Wasser-Emulsion (O/W-Emulsion) vorliegt.

In einem solchen Fall sind die erfindungsgemäß vorteilhaften Ausführungsformen der vorliegenden Erfindung dadurch gekennzeichnet, dass die Zubereitung einen oder mehrere Emulgatoren gewählt aus der Gruppe der Verbindungen Glycerylstearatcitrat, Cetearylalkohol, Natriumcetearylsulfat, Glycerylstearat, Cetearylsulfosuccinat Natriumstearoylglutamat, Polyglyceryl-3-methylglucosedistearat Polyglyceryl-3-methylglucosedistearat, Stearinsäure, Kaliumcetylphosphat, enthalten.

Es ist erfindungsgemäß vorteilhaft, wenn die erfindungsgemäße Zubereitung einen oder mehrere UV-Filterenthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetrame thylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)-ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4-(tert.-Butyl)-4'-methoxydibenzoylmethan; 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist dabei erfindungsgemäß besonders bevorzugt, wenn die Zubereitung frei ist von 3-(4-Methylbenzyliden)-campher und 2-Hydroxy-4-methoxybenzophenon (Oxybenzon).

Erfindungsgemäß vorteilhafte Ausführungsformen können dadurch erhalten werden, dass die Zubereitung Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Es ist erfindungsgemäß vorteilhaft, wenn die erfindungsgemäße Zubereitung Phenoxyethanol und/oder Methylparaben enthält.

Es ist dabei erfindungsgemäß bevorzugt, wenn die Zubereitung frei ist von Propyl- und Butylparaben, 3-lod-2-propinylbutylcarbamat,

Erfindungsgemäß vorteilhafte Ausführungsformen sind auch dadurch gekennzeichnet, dass die Zubereitung einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

Die erfindungsgemäße Zubereitung kann ferner vorteilshaft Glycerin und/oder Ethanol enthalten. In einem solchen Falle ist eine Glycerin-Konzentration von 0,01 bis 10 Gewichts-%, bezogen auf das Gesamtgewicht der Zubereitung, erfindungsgemäß vorteilhaft. Für Ethanol liegt der erfindungsgemäß vorteilhafte Einsatzbereich zwischen 0,01 und 10 Gewichts-%, bezogen auf das Gesamtgewicht der Zubereitung.

Die Ölphase der erfindungsgemäßen Zubereitung kann darüber hinaus noch weitere Öl-, Fett- und Wachskomponenten enthalten, beispielsweise polaren Öle aus der Gruppe der Lecithine oder Verbindungen wie z. B. Cocoglycerid, Capryl/Caprinsäure Triglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyl-lactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂₋₁₅-Alkylbenzoat aufweist.

Vorteilhafte Ölkomponenten sind ferner z. B. Isopropylpalmitat, Myristylmyristat, Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Erfindungsgemäß vorteilhafte Ausführungsformen der vorliegenden Erfindung sind auch dadurch gekennzeichnet, dass die Emulsion Dimethicone und/oder Cyclomethicon enthält.

Als polymeren Filmbildner zur Erhöhung der Wasserfestigkeit kann die erfindungsgemäße Zubereitung erfindungsgemäß vorteilhaft Vinylpyrrolidon/Hexadecen Copolymer enthalten. Darüber hinaus ist der Zusatz Tapiokastärke erfindungsgemäß vorteilhaft.

Die erfindungsgemäße Zubereitung kann insbesondere vorteilhaft als Tagespflegeprodukt oder Sonnenschutzmittel eingesetzt werden.

Erfindungsgemäß ist auch ein Verfahren zur Erleichterung der Auswaschbarkeit von 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthaltenden kosmetischen Zubereitungen aus Textilien, welches dadurch gekennzeichnet ist, dass dem Kosmetikum ein oder mehrere erfindungsgemäße Komplexbildner zugesetzt werden.

Auch ist das Verfahren zur Reduzierung der durch UV-Lichtschutzfilter (insbesondere 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine)) enthaltenden kosmetischen Zubereitungen hervorgerufenen Textilverfleckung, welches dadurch gekennzeichnet ist, dass dem Kosmetikum ein oder mehrere Komplexbildner zugesetzt werden, erfindungsgemäß.

Erfindungsgemäß ist ferner die Verwendung von erfindungsgemäßen Komplexbildnern in 2,4-Bis-{[4-(2-ethylhexyloxy)-2-hydroxy]-phenyl}-6-(4-methoxyphenyl)-1,3,5-triazin (INCI Bis-Ethylhexyloxyphenol Methoxyphenyl Triazine) enthaltenden kosmetischen Zubereitungen zur Erleichterung der Auswaschbarkeit der UV-Lichtschutzfilter aus mit den Zubereitungen kontaminierten Textilien, sowie die Verwendung von erfindungsgemäßen Komplexbildnern in UV-Lichtschutzfilter (insbesondere 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine)) enthaltenden kosmetischen Zubereitungen zur Reduzierung der durch die Zubereitung hervorgerufene Textilverfleckung.

### Vergleichsversuch

Mit dem folgenden Versuch konnte der erfindungsgemäße Effekt beispielhaft belegt werden: Es wurde jeweils 1% der erfindungsgemäßen Hilfsstoffe zu einer Bis-Ethylhexyloxyphenol Methoxyphenyl Triazine enthaltenden Formulierung zugesetzt und die Verfleckungsreduzierende Wirkung (Reduktion b*) im Vergleich zu einer Formulierung ohne erfindungsgemäße Komplexbildner mittels beschriebener Methode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in der Abbildung 1 und Tabelle 1 dargestellt sind.

Es wurden verschiedene Sonnenschutzemulsionen hinsichtlich der Bildung von gelben Flecke über einen in vitro Auftragungs-/ Waschzyklus untersucht. Es wurden dabei weiße vorgewaschene Baumwollmonitore (100% Baumwolle) verwendet. Dazu wurden je 25 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, zehn Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: getestete Zubereitungen und deren Gelbwert-Reduktion von Flecken; b*-Wert [%] Beispiele 1, 2, 5, 7 und 9 sind Referenzbeispiele**

| **INCI** | **Bsp.1** | **Bsp.2** | **Bsp.3** | **Bsp.4** | **Bsp.5** | **Bsp.6** | **Bsp.7** |
|---|---|---|---|---|---|---|---|
| Potassium Trisphosphonomethylamine Oxide | 1,0 | | | | | | |
| Natriumpolyphosphat | | 1,0 | | | | | |
| Tetranatrium Pyrophosphat | | | 1,0 | | | | |
| Natriumdiethylenetriaminepentamethylenephosphonate | | | | 1,0 | | | |
| Natrium EDTMP | | | | | 1,0 | | |
| Diethylentriaminpentamethylenphosphonsäure/ Salzsäure | | | | | | 1,0 | |
| Caprylic/Capric Triglyceride | 4,0 | 4,0 | 4,0 | 4,0 | 4,0 | 4,0 | 4,0 |
| Glyceryl Stearate | 1,0 | 1,0 | 1,0 | 1,0 | 1,0 | 1,0 | 1,0 |
| Hydrogenated Coco-Glycerides | 1,0 | 1,0 | 1,0 | 1,0 | 1,0 | 1,0 | 1,0 |
| Sodium Stearoyl Glutamate | 0,3 | 0,3 | 0,3 | 0,3 | 0,3 | 0,3 | 0,3 |
| Silica Dimethyl Silylate | 0,3 | 0,3 | 0,3 | 0,3 | 0,3 | 0,3 | 0,3 |
| Tapioka Stärke | 1,0 | 1,0 | 1,0 | 1,0 | 1,0 | 1,0 | 1,0 |
| Parfum | 0,5 | 0,5 | 0,5 | 0,5 | 0,5 | 0,5 | 0,5 |
| Glycerin | 0,9 | 0,9 | 0,9 | 0,9 | 0,9 | 0,9 | 0,9 |
| Phenoxyethanol | 0,6 | 0,6 | 0,6 | 0,6 | 0,6 | 0,6 | 0,6 |
| Methylparaben | 0,3 | 0,3 | 0,3 | 0,3 | 0,3 | 0,3 | 0,3 |
| Acrylates/C10-30 Alkyl Acrylate Crosspolymer | 0,15 | 0,15 | 0,15 | 0,15 | 0,15 | 0,15 | 0,15 |
| Xanthan Gummi | 0,4 | 0,4 | 0,4 | 0,4 | 0,4 | 0,4 | 0,4 |
| Alcohol Denat. | 8,0 | 8,0 | 8,0 | 8,0 | 8,0 | 8,0 | 8,0 |
| Trisnatrium EDTA | 0,2 | 0,2 | 0,2 | 0,2 | 0,2 | 0,2 | 0,2 |
| Octocrylene | 9,0 | 9,0 | 9,0 | 9,0 | 9,0 | 9,0 | 9,0 |
| Bis-Ethylhexyloxyphenol Methoxyphenyl Triazine | 3,0 | 3,0 | 3,0 | 3,0 | 3,0 | 3,0 | 3,0 |
| Wasser | ad. 100 | ad. 100 | ad. 100 | ad. 100 | ad. 100 | ad. 100 | ad. 100 |
| | | | | | | | |
| Reduktion b* [%] | -31 | -45 | -23 | -87 | -84 | -85 | 0 |

Die Ergebnisse zeigen eine eindeutige Fleckenverminderung durch den Einsatz von Komplexbildnern im Vergleich zur Formulierung ohne die erfindungsgemäßen Komplexbildner (Beispiel 7).

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **Beispiel [%]** | | | | |
|---|---|---|---|---|---|
| | **8** | **9** | **10** | **11** | **12** |
| Potassium Trisphosphonomethylamine Oxide | | 0,50 | | | 0,50 |
| Natriumpolyphosphat | | | 0,50 | | |
| Tetranatrium Pyrophosphat | | | | 0,50 | |
| Natriumdiethylenetriaminepentamethylenephosphonate | 0,50 | | | | 0,50 |
| Natrium EDTMP | | 0,50 | | | |
| Diethylentriaminpentamethylenphosphonsäure/ Salzsäure | | | 0,50 | | |
| Trisodium EDTA | 0,20 | 0,50 | 0,50 | 0,20 | 0,50 |
| Butyl methoxydibenzoylmethane | 5,00 | 3,00 | 3,00 | 5,00 | 4,50 |
| Bis-Ethylhexyloxyphenol Methoxyphenyl Triazine | 3,50 | 3,50 | 5,50 | 5,50 | 3,50 |
| Phenylbenzimidazole Sulfonic Acid | | 1,00 | 1,00 | 1,00 | 1,00 |
| Ethylhexyl Salicylate | | 5,00 | 5,00 | 5,00 | |
| Titanium Dioxide | 3,00 | 2,00 | | | 3,00 |
| Trimethoxycaprylylsilane | 0,20 | 0,20 | | | 0,20 |
| Octocrylene | 10,00 | 10,00 | 10,00 | 10,00 | 9,00 |
| Homosalate | | 10,00 | 10,00 | 10,00 | 9,50 |
| Cetearyl Alcohol | | 1,00 | 0,50 | | |
| Xanthan Gum | 0,40 | 0,40 | 0,40 | | 0,40 |
| Acrylates/C10-30 Alkyl Acrylate Crosspolymer | 0,30 | 0,05 | 0,20 | 0,40 | 0,30 |
| Alcohol Denat. | 5,00 | 4,00 | 6,00 | 6,00 | 5,00 |
| Methylparaben | 0,30 | 0,30 | 0,30 | 0,30 | 0,30 |
| Phenoxyethanol | 0,60 | 0,60 | 0,60 | 0,60 | 0,60 |
| Citric Acid | 0,30 | | | | 0,30 |
| Sodium Citrate | 0,10 | | | | 0,10 |
| Glycerin | 3,00 | 9,00 | 3,00 | 9,00 | 3,00 |
| Parfum | | 0,40 | 0,60 | 0,30 | |
| VP/Hexadecene Copolymer | 0,50 | 0,50 | | 0,50 | 0,50 |
| Silica Dimethyl Silylate | | 0,50 | 0,50 | | |
| Sodium Cetearyl Sulfate | | 0,15 | | | |
| Glyceryl Stearate SE | | 1,00 | | | |
| Glyceryl Stearate Citrate | 2,00 | | | | 2,00 |
| Ceteareth-20 | | | | 1,00 | |
| Sodium Stearoyl Glutamate | | | 0,40 | | |
| Glyceryl Stearate | | | 1,00 | | |
| Hydrogenated Coco-Glycerides | 1,00 | | 1,00 | | 1,00 |
| C12-15 Alkyl Benzoate | 5,00 | | | | 5,00 |
| Myristyl Myristate | 1,00 | 1,00 | | | 1,00 |
| Stearyl Alcohol | 0,50 | | | | 0,50 |
| C18-36 Acid Triglyceride | | | | 0,50 | |
| C18-38 Alkyl Hydroxystearoyl Stearate | | 0,50 | | | |
| Isopropyl Stearate | 2,00 | | | | 2,00 |
| Butylene Glycol Dicaprylate/Dicaprate | 5,00 | | | 3,00 | 5,00 |
| Aqua | ad 100 | ad 100 | ad 100 | ad 100 | ad 100 |

## Patentansprüche

1. Kosmetische Zubereitung enthaltend
a) 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) und
b) einen oder mehrere Komplexbildner gewählt aus der Gruppe
- Aminotrimethylenphosphonsäure/ ATMP
- Diethylentriaminpenta(methylenphosphonsäure)/ DTPMP
- Tetranatriumpyrophosphat
- und/oder deren Alkalisalze.

2. Kosmetische Zubereitung nach Anspruch 1, **dadurch gekennzeichnet, dass** die Zubereitung a) 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), in einer Menge von 0,01 bis 10,0 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung in Form einer Öl-in -Wasser-Emulsion (O/W-Emulsion) vorliegt.

4. Kosmetische Zubereitung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Cetearylalkohol Natriumcetearylsulfat + Glycerylstearat, Cetearylsulfosuccinat Natriumstearoylglutamat, Polyglyceryl-3-methylglucosedistearat Polyglyceryl-3-methylglucosedistearat, Stearinsäure, Kaliumcetylphosphat, enthält.

5. Kosmetische Zubereitung nach einem der vorhergehenden Ansprüche, **dadurch gekennzeichnet, dass** die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4-(tert.-Butyl)-4'-methoxydibenzoylmethan; 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

6. Kosmetische Zubereitung nach einem der vorhergehenden Ansprüche, **dadurch gekennzeichnet, dass** die Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

7. Kosmetische Zubereitung nach einem der vorhergehenden Ansprüche, **dadurch gekennzeichnet, dass** die Zubereitung Phenoxyethanol und/oder Methylparaben enthält.

8. Kosmetische Zubereitung nach einem der vorhergehenden Ansprüche, **dadurch gekennzeichnet, dass** die Emulsion einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

9. Verfahren zur Erleichterung der Auswaschbarkeit von 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen aus Textilien, **dadurch gekennzeichnet, dass** dem Kosmetikum ein oder mehrere Komplexbildner aus der Gruppe
- Aminotrimethylenphosphonsäure/ ATMP
- Diethylentriaminpenta(methylenphosphonsäure)/ DTPMP
- Tetranatriumpyrophosphat
und/oder deren Alkalisalze zugesetzt werden.

10. Verwendung von Komplexbildnern aus der Gruppe
- Aminotrimethylenphosphonsäure/ ATMP
- Diethylentriaminpenta(methylenphosphonsäure)/ DTPMP
- Tetranatriumpyrophosphat
und/oder deren Alkalisalze
in 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), enthaltenden kosmetischen Zubereitungen zur Erleichterung der Auswaschbarkeit der UV-Lichtschutzfilter aus mit den Zubereitungen kontaminierten Textilien.

## Claims

1. Cosmetic preparation comprising
a) 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) and
b) one or more chelating agents selected from the group
- aminotrimethylenephosphonic acid/ ATMP
- diethylenetriaminepenta(methylenephosphonic acid)/ DTPMP
- tetrasodium pyrophosphate
- and/or alkali metal salts thereof.

2. Cosmetic preparation according to Claim 1, **characterized in that** the preparation comprises a) 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) in an amount of 0.01 to 10.0% by weight, based on the total weight of the preparation.

3. Cosmetic preparation according to either of the preceding claims, **characterized in that** the preparation is in the form of an oil-in-water emulsion (O/W emulsion).

4. Cosmetic preparation according to any of the preceding claims, **characterized in that** the preparation comprises one or more emulsifiers selected from the group of compounds comprising glyceryl stearate citrate, cetearyl alcohol, sodium cetearyl sulfate + glyceryl stearate, cetearyl sulfosuccinate, sodium stearoyl glutamate, polyglyceryl-3 methylglucose distearate, polyglyceryl-3 methylglucose distearate, stearic acid, potassium cetyl phosphate.

5. Cosmetic preparation according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 4-(tert-butyl)-4'-methoxydibenzoylmethane; tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

6. Cosmetic preparation according to any of the preceding claims, **characterized in that** the preparation comprises one or more compounds selected from the group of compounds comprising ethylhexylglycerin, polyglyceryl-2 caprate, propylene glycol, butylene glycol, 2-methylpropane-1,3-diol, 1,2-pentanediol, 1,2-hexanediol, 1,2-octanediol and/or 1,2-decanediol.

7. Cosmetic preparation according to any of the preceding claims, **characterized in that** the preparation comprises phenoxyethanol and/or methylparaben.

8. Cosmetic preparation according to any of the preceding claims, **characterized in that** the emulsion comprises one or more active ingredients selected from the group of compounds comprising magnolia extract, gylcyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine and/or licochalcone A.

9. Method for facilitating the ability of cosmetic preparations comprising 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) to be washed out from textiles, **characterized in that** one or more chelating agents agents from the group
- aminotrimethylenephosphonic acid/ ATMP
- diethylenetriaminepenta(methylenephosphonic acid)/ DTPMP
- tetrasodium pyrophosphate
and/or alkali metal salts thereof, are added to the cosmetic.

10. Use of chelating agents from the group
- aminotrimethylenephosphonic acid/ ATMP
- diethylenetriaminepenta(methylenephosphonic
acid)/ DTPMP
- tetrasodium pyrophosphate
and/or alkali metal salts thereof
in cosmetic preparations comprising 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) for facilitating the ability of UV photoprotective filters to be washed out from textiles contaminated with the preparations.

## Revendications

1. Préparation cosmétique contenant :
a) de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) et
b) un ou plusieurs complexants choisis dans le groupe composé par
- l'acide aminotriméthylène-phosphonique/ ATMP,
- l'acide diéthylène-triamine-penta(méthylène-phosphonique)/ DTPMP,
- le pyrophosphate tétrasodique,
- et/ou leurs sels alcalins.

2. Préparation cosmétique selon la revendication 1, **caractérisée en ce que** la préparation contient a) de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) en une quantité de 0,01 à 10,0 % en poids, par rapport au poids total de la préparation.

3. Préparation cosmétique selon l'une quelconque des revendications précédentes, **caractérisée en ce que** la préparation se présente sous la forme d'une émulsion huile-dans-eau (émulsion H/E).

4. Préparation cosmétique selon l'une quelconque des revendications précédentes, **caractérisée en ce que** la préparation contient un ou plusieurs émulsifiants choisis dans le groupe de composés composé par le stéarate-citrate de glycéryle, l'alcool cétéarylique, le sulfate de cétéaryle sodium + stéarate de glycéryle, le sulfosuccinate de cétéaryle, le glutamate de stéaroyle sodium, le distéarate de polyglycéryl-3-méthylglucose, le distéarate de polyglycéryl-3-méthylglucose, l'acide stéarique, le phosphate de cétyle potassium.

5. Préparation cosmétique selon l'une quelconque des revendications précédentes, **caractérisée en ce que** la préparation contient un ou plusieurs filtres UV, qui sont choisis dans le groupe de composés composé par : l'acide 2-phénylbenzimidazole-5-sulfonique et/ou ses sels, les sels de l'acide phénylène-1,4-bis-(2-benzimidazyl)-3,3'-5,5'-tétrasulfonique, le 1,4-di(2-oxo-10-sulfo-3-bornylidène-méthyl)-benzène et ses sels ; les sels de l'acide 4-(2-oxo-3-bornylidène-méthyl)benzène-sulfonique ; les sels de l'acide 2-méthyl-5-(2-oxo-3-bornylidène-méthyl)sulfonique ; le 2,2'-méthylène-bis-(6-(2H-benzotriazol-2-yl)-4-(1,1,3,3-tétraméthylbutyl)-phénol) ; le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; le 3-(4-méthylbenzylidène)camphre ; le 3-benzylidène-camphre ; le salicylate d'éthylhexyle ; l'acide téréphtalidène-dicamphre-sulfonique ; le 2-cyano-3,3-diphenylacrylate de 2-éthylhexyle ; l'ester 2-éthylhexylique de l'acide 4-(diméthylamino)-benzoïque ; l'ester amylique de l'acide 4-(diméthylamino)benzoïque ; l'ester di(2-éthylhexylique) de l'acide 4-méthoxybenzalmalonique ; l'ester 2-éthylhexylique de l'acide 4-méthoxycinnamique ; l'ester isoamylique de l'acide 4-méthoxycinnamique ; la 2-hydroxy-4-méthoxybenzophénone, la 2-hydroxy-4-méthoxy-4'-méthylbenzophénone ; la 2,2'-dihydroxy-4-méthoxybenzophénone ; le salicylate d'homomenthyle ; le 2-hydroxybenzoate de 2-éthylhexyle ; le benzalmalonate de diméthicodiéthyle ; le copolymère de 3-(4-(2,2-bis-éthoxycarbonylvinyl)-phénoxy)propényl)-méthoxysiloxane/diméthylsiloxane ; l'ester hexylique de l'acide 2-(4'-diéthylamino-2'-hydroxybenzoyl)-benzoïque ; la dioctylbutylamidotriazone (INCI : Diethylhexyl-Butamidotriazone) ; la 2,4-bis-[5-1-(diméthylpropyl)benzoxazol-2-yl-(4-phényl)-imino]-6-(2-éthylhexyl)-imino-1,3,5-triazine de n°CAS 288254-16-0) ; la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) ; le 4-(tert.butyl)-4'-méthoxydibenzoylméthane ; l'ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine (INCI : Ethylhexyl Triazone) ; la 2,4,6-tribiphényl-4-yl-1,3,5-triazine ; la mérocyanine ; le dioxyde de titane ; l'oxyde de zinc.

6. Préparation cosmétique selon l'une quelconque des revendications précédentes, **caractérisée en ce que** la préparation contient un ou plusieurs composés choisis dans le groupe de composés composé par l'éthylhexylglycérine, le 2-caprate de polyglycéryle, le propylène glycol, le butylène glycol, le 2-méthylpropane-1,3-diol, le 1,2-pentanediol, le 1,2-hexanediol, le 1,2-octanediol et/ou le 1,2-décanediol.

7. Préparation cosmétique selon l'une quelconque des revendications précédentes, **caractérisée en ce que** la préparation contient du phénoxyéthanol et/ou du méthylparabène.

8. Préparation cosmétique selon l'une quelconque des revendications précédentes, **caractérisée en ce que** l'émulsion contient un ou plusieurs agents actifs choisis dans le groupe de composés composé par l'extrait de magnolia, l'acide glycyrrhizique, l'urée, l'arctiine, l'acide alpha-liponique, l'acide folique, le phytoène, la D-biotine, la coenzyme Q10, l'alpha-glucosylrutine, la carnitine, la carnosine, la caféine, les isoflavonoïdes naturels et/ou synthétiques, le glycérylglucose, la créatine, la créatinine, la taurine, le tocophérol, l'acétate de tocophérol, la β-alanine et/ou la licochalcone A.

9. Procédé visant à faciliter la rinçabilité de préparations cosmétiques contenant de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) à partir de textiles, **caractérisé en ce qu'**un ou plusieurs complexants du groupe composé par :
- l'acide aminotriméthylène-phosphonique/ ATMP,
- l'acide diéthylène-triamine-penta(méthylène-phosphonique)/ DTPMP,
- le pyrophosphate tétrasodique,
et/ou leurs sels alcalins, sont ajoutés au cosmétique.

10. Utilisation de complexants du groupe composé par :
- l'acide aminotriméthylène-phosphonique/ ATMP,
- l'acide diéthylène-triamine-penta(méthylène-phosphonique)/ DTPMP,
- le pyrophosphate tétrasodique,
et/ou leurs sels alcalins,
dans des préparations cosmétiques contenant de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) pour faciliter la rinçabilité des filtres de protection contre la lumière UV à partir de textiles contaminés par les préparations.
